# EUROPEAN PATENT APPLICATION

(11) **EP 1 523 976 A2**
(43) Date of publication of application: **20.04.2005**
(21) Application number: 05075040.5
(22) Date of filing: 23.11.2000
(51) Int. Cl.: A61K 9/12, A61K 31/137, A61P 11/06

(54) **Pharmaceutical formulations of salmeterol**

(30) Priority: 23.11.1999 GB 9927685; 24.12.1999 GB 9930700; 13.06.2000 GB 0014424
(62) Divisional of application: 00977707.9
(71) Applicant: GLAXO GROUP LIMITED, Greenford, Middlesex UB6 0NN (GB)
(72) Inventor: Cripps, Alan Leslie, c/o GlaxoSmithKline, Hertfordshire SG12 0DP (GB); Johnson, Paul, c/o GlaxoSmithKline, Hertfordshire SG12 0DP (GB)
(74) Representative: Teuten, Andrew John

(57) **Abstract**

There is provided according to the invention a pharmaceutical aerosol formulation which comprises:
(i) salmeterol or a pharmaceutically acceptable salt thereof and
(ii) a hydrofluoroalkane (HFA) propellant,
characterised in that the salmeterol or pharmaceutically acceptable salt thereof is completely dissolved in the formulation.

## Description

### Background of the Invention

### Field of the invention

The present invention relates to a pharmaceutical formulation for use in the administration of medicaments by inhalation. In particular, this invention relates to a pharmaceutical formulation of salmeterol or a pharmaceutically acceptable salt thereof (such as the xinafoate salt) for use in pressurised metered dose inhalers (MDI's). The invention also relates to methods for their preparation and to their use in therapy.

### Description of the background art

Inhalers are well known devices for administering pharmaceutically active materials to the respiratory tract by inhalation. Such active materials commonly delivered by inhalation include bronchodilators such as β2 agonists and anticholinergics, corticosteroids, anti-allergies and other materials that may be efficiently administered by inhalation, thus increasing the therapeutic index and reducing side effects of the active material.

4-hydroxy-α¹-[[[6-(4-phenylbutoxy)hexyl]amino]methyl]-1,3-benzenedimethanol was described as one of a wide range of bronchodilators in GB-A-2140800. This compound is also known by the generic name of salmeterol, the 1-hydroxy-2-naphthoate (xinafoate) salt of which has become widely known as a highly effective treatment of inflammatory diseases, such as asthma and chronic obstructive pulmonary disease (COPD).

Metered dose inhalers (MDI's) are the most common type of a wide range of inhaler types and utilise a liquefied propellant to expel droplets containing the pharmaceutical product to the respiratory tract as an aerosol. MDI formulations are generally characterised as solution formulations or suspension formulations.

The most commonly used aerosol propellants for medicaments have been Freon 11 (CCl₃F) in admixture with Freon 12 (CCl₂F₂) and Freon 114 (CF₂Cl.CF₂Cl). However, these propellants are now believed to provoke the degradation of stratospheric ozone and their use is now being phased out to eliminate the use of all CFC containing aerosol propellants. There is thus a need to provide an aerosol formulation for medicaments which employ so called 'ozone-friendly' propellants.

Hydrofluoroalkanes (HFAs; known also as hydrofluorocarbons or HFCs) contain no chlorine and are considered less destructive to ozone and these are proposed substitutes for CFCs. In particular, 1,1,1,2-tetrafluoroethane (HFA 134a) and 1,1,1,2,3,3,3-heptafluoropropane (HFA 227) have been acknowledged to be the best candidates for non-CFC propellants.

The efficiency of an aerosol device, such as an MDI, is a function of the dose deposited at the appropriate site in the lungs. Deposition is affected by several factors, of which one of the most important is the aerodynamic particle size. Solid particles and/or droplets in an aerosol formulation can be characterised by their mass median aerodynamic diameter (MMAD, the diameter around which the mass aerodynamic diameters are distributed equally).

Particle deposition in the lung depends largely upon three physical mechanisms:
1. impaction, a function of particle inertia;
2. sedimentation due to gravity; and
3. diffusion resulting from Brownian motion of fine, submicrometer (<1µm) particles.

The mass of the particles determines which of the three main mechanisms predominates.

The effective aerodynamic diameter is a function of the size, shape and density of the particles and will affect the magnitude of forces acting on them. For example, while inertial and gravitational effects increase with increasing particle size and particle density, the displacements produced by diffusion decrease. In practice, diffusion plays little part in deposition from pharmaceutical aerosols. Impaction and sedimentation can be assessed from a measurement of the MMAD which determines the displacement across streamlines under the influence of inertia and gravity, respectively.

Aerosol particles of equivalent MMAD and GSD (geometric standard deviation) have similar deposition in the lung irrespective of their composition. The GSD is a measure of the variability of the aerodynamic particle diameters.

For inhalation therapy there is a preference for aerosols in which the particles for inhalation have a diameter of about 0.5 to 5µm. Particles which are larger than 5µm in diameter are primarily deposited by inertial impaction in the orthopharynx, particles 0.5 to 5µm in diameter, influenced mainly by gravity, are ideal for deposition in the conducting airways, and particles 0.5 to 3µm in diameter are desirable for aerosol delivery to the lung periphery. Particles smaller than 0.5µm may be exhaled.

Respirable particles are generally considered to be those with aerodynamic diameters less than 5µm. These particles, particularly those with a diameter of about 3µm, are efficiently deposited in the lower respiratory tract by sedimentation.

It has been recently demonstrated in patients with mild and severe airflow obstruction that the particle size of choice for a β2 agonist or anticholinergic aerosol should be approximately 3µm (Zaanen, P. et al, Int. J. Pharm. (1994) 107, 211-217, Int. J. Pharm. (1995) 114, 111-115, Thorax (1996), 51, 977-980.)

Many of the factors relevant to the MMAD of particles are relevant to droplets and the additional factors of rate of solvent evaporation and surface tension are also important.

In suspension formulations, particle size in principle is controlled during manufacture by the size to which the solid medicament is reduced, usually by micronisation.
However, if the suspended drug has the slightest solubility in propellant, a process known as Ostwald Ripening can lead to particle size growth. Also, particles may have tendency to aggregate, or adhere to parts of the MDI eg. canister or valve. The effect of Ostwald ripening and particularly of drug deposition may be particularly severe for potent drugs (including salmeterol) which need to be formulated in low doses. Solution formulations do not suffer from these disadvantages, but suffer from different ones in that particle size is both a function of rate of evaporation of the propellant from the formulation, and of the time between release of formulation from the canister and the moment of inhalation. Thus, it may be subject to considerable variability and is generally hard to control.

Besides its impact on the therapeutic profile of a drug, the size of aerosol particles has an important impact on the side effect profile of a drug. For example, it is well known that the orthopharynx deposition of aerosol formulations of steroids can result in side effects such as candidiasis of mouth and throat. Furthermore, a higher systemic exposure to the aerosol particles due to deep lung penetration can enhance the undesired systemic effects of certain drugs. For example, the systemic exposure to certain steroids can produce side effects on bone metabolism and growth.

We have now invented a formulation of salmeterol which eliminates or substantially mitigates some or all of the above mentioned disadvantages.

### Summary of the invention

Thus, according to the present invention we provide a pharmaceutical aerosol formulation, comprising (i) salmeterol or a pharmaceutically acceptable salt thereof and (ii) a hydrofluoroalkane (HFA) propellant; and characterised in that the salmeterol or pharmaceutically acceptable salt thereof is completely dissolved in the formulation.

### Detailed description of the invention

The formulation will generally contain a solubilisation agent to aid solubilisation of the salmeterol or pharmaceutically acceptable salt in the formulation. Suitable solubilisation agents include propylene glycol and ethanol, preferably ethanol. Other suitable solubilisation agents include alkanes and ethers (eg dimethyl ether). A further solubilisation agent of interest is dimethoxymethane. Other potential solubilising agents include propan-1-ol, propan-2-ol, ethyl acetate and polyethylene glycol (eg PEG200, PEG400).

As a particular aspect of the present invention we provide a pharmaceutical aerosol formulation, comprising (i) salmeterol or a pharmaceutically acceptable salt thereof, (ii) a hydrofluoroalkane (HFA) propellant, (iii) a low volatility component to increase the mass median aerodynamic diameter (MMAD) of the aerosol particles on actuation of the inhaler and (iv) a solubilisation agent in sufficient quantity to solubilise the salmeterol or pharmaceutically acceptable salt thereof in the formulation.

The presence of the low volatility component in the solution formulation increases the fine particle mass (FPM) as defined by the content of stages 3-5 of an Andersen Cascade Impactor on actuation of the formulation relative to solutions formulations which omit this component. Solution formulations which omit the higher volatility component generally give rise to a particle size distribution which have a higher content of finer particles; such distributions generally do not match the distribution of the existing commercialised suspension formulations which contain CFC's and may therefore not be bio-equivalent.

Examples of HFA propellants include 1,1,1,2-tetrafluoroethane (HFA134a) and 1,1,1,2,3,3,3-heptaf)uoro-n-propane (HFA227) and mixtures thereof. The preferred propellant is 1,1,1,2-tetrafluoroethane (HFA134a). An alternative propellant of interest is 1,1,1,2,3,3,3-heptafluoro-n-propane (HFA227).

The preferred low volatility component is glycerol, propylene glycol or polyethyleneglycol (eg PEG200 or PEG400). Glycerol is of particular interest. Polyethyleneglycol is also of particular interest eg PEG200 or PEG400 especially PEG200. Preferably the low volatility component is present in an amount of 0.5 to 3% (w/w).

The preferred solubilisation agent is ethanol.

In a first embodiment of the invention we prefer salmeterol to be used in the form of the xinafoate salt. Salmeterol xinafoate may be prepared in two polymorphic forms known as Form I and Form II. Form which has a melting endotherm at 140 °C may be prepared by precipitation from a hot methanolic solution of salmeterol xinafoate on addition to cold isopropanol as described in International Patent Application No. WO93/16031. Form II which has a melting endotherm at 125 °C may be prepared by supercritical fluid recrystallisation as described in International Patent Application No. WO95/01324. Preferably salmeterol xinafoate is employed as Form II polymorph since this form would be predicted to have a higher solubility. Alternatively salmeterol xinafoate may be employed as the Form I polymorph.

More particularly we prefer to use salmeterol xinafoate in the form of the purified enantiomer R-salmeterol xinafoate. Surprisingly we have found that R-salmeterol xinafoate in a polymorphic form obtainable by crystallisation from ether is significantly more soluble in mixtures of ethanoI/HFA134a and ethanol/HFA227 than racemic salmeterol xinafoate. Without being limited by theory, this higher solubility may be attributed to the low crystal lattice energy as demonstrated by a melting endotherm at 95 °C (which is considerably lower than that of the two forms of salmeterol xinafoate mentioned above).

In a second embodiment of the invention we prefer to use salmeterol as the free base.

Surprisingly we have found that salmeterol base is substantially more soluble in mixtures of ethanol/HFA134a and ethanol/HFA227 than racemic salmeterol xinafoate or even R-salmeterol xinafoate. It is also of interest to use salmeterol base as R-salmeterol base.

Use of R-salmeterol xinafoate or base has the further advantage that it takes advantage of the higher potency of R-salmeterol relative to racemic salmeterol with the result that a lower concentration of the drug in solution is required.

In a third less preferred embodiment salmeterol is used as the sulphate salt. The preferred solubilising agent for salmeterol sulphate is propylene glycol.

As is apparent from the examples, formulations of salmeterol base in ethanol and HFA134a or HFA227 show particularly excellent delivery characteristics and closely reproduce the particle distribution properties of the currently marketed CFC-containing suspension formulation of salmeterol xinafoate.

In the foregoing, except where otherwise indicated, drug quantities are given as appropriate for salmeterol base but it will be understood that for a salmeterol xinafoate or another pharmaceutically acceptable salt thereof an appropriate conversion to give a suitable weight of active principle in the delivered dose may be made. For example a dose of 25 µg of salmeterol equates to a dose of 36.3 µg of salmeterol xinafoate. It will also be understood that salmeterol may be used as the racemate or in the form of an enantiomerically enriched (or purified) single R- or S- enantiomer. In the foregoing drug quantities are given as appropriate for racemic drug but it will be understood that adjustment of the dosage weight may be appropriate when a different ratio of enantiomers is employed. For example R-salmeterol may desirably be employed at one half of the normal dose of racemic salmeterol.

We prefer the formulation to be suitable for delivering a therapeutic amount of salmeterol (eg as xinafoate) in one or two actuations. Preferably the formulation will be suitable for delivering 25-50 µg salmeterol per actuation, especially 25 µg per actuation.

The formulation according to the invention will be used in association with a suitable metering valve. We prefer that the formulation is actuated by a metering valve capable of delivering a volume of between 50µl and 100µl, eg 50µl or 63µl. 100µl is also suitable. For a 25 µg dose, when a 50µl metering volume is used, the final concentration of salmeterol delivered per actuation would be 0.05% (w/v) or 0.042% (w/w). Wherein a 63µl metering volume is used, the final concentration of salmeterol delivered per actuation would be 0.04% (w/v) or 0.033% (w/w). If a 100 µl metering valve were to be used, for a 25 µg dose the final concentration of salmeterol delivered per actuation would be 0.025% (w/v) or 0.021% (w/w). The previously referred to w/w figures are approximate in that they do not compensate for the density mismatch between HFA134a and ethanol, however the precise figures may be readily determined.

Use of a larger metering chamber eg 100 µl is generally preferred.

We prefer the formulation to contain between 0.8 and 1.6 % w/w, particularly 1.0 and 1.6% (w/w) of a low volatility component. We especially prefer to use 1.3% (w/w). We also especially prefer to use 1.0% (w/w) of the low volatility component. However the most preferred range for the low volatility component is 0.5-1 % w/w eg 0.5%, 0.75% or 1% w/w.

It is necessary to employ the low volatility component, the solubilising agent and the propellant in relative proportions such that the components are freely miscible.

Depending on the final concentration of salmeterol or pharmaceutically acceptable salt thereof in the formulation, the propellant, and the precise amount of low volatility component, the concentration of solubilising agent (eg ethanol) required will vary. So as not to suppress the vapour pressure of the propellant to an undesirable extent, the amount of ethanol should preferably not exceed around 40%, more preferably 35%. The amount of ethanol will more preferably be in the range 5 to 40%, especially 5 to 30% eg 13 to 24%. The concentration of salmeterol expressed as weight of xinafoate will typically be in the range 0.02-0.05% w/v.

When the concentration of salmeterol xinafoate is around 0.05% w/v and the propellant is 1,1,1,2-tetrafluoroethane, an amount of ethanol of 28-32% w/w, especially around 30% w/w is particularly suitable. When the concentration of salmeterol (present as xinafoate) is around 0.05% w/v (based on weight of salmeterol base) and the propellant is 1,1,1,2-tetrafluoroethane, an amount of ethanol of 46-49% w/w is suitable eg 48% w/w. When the concentration of salmeterol xinafoate is around 0.04% w/v and the propellant is 1,1,1,2-tetrafluoroethane, an amount of ethanol of 22-26% w/w, especially around 24% w/w is particularly suitable. When the concentration of salmeterol (present as xinafoate) is around 0.04% w/v (based on weight of salmeterol base) and the propellant is 1,1,1,2-tetrafluoroethane, the amount of ethanol is preferably 35-38% w/w eg 37% w/w. When the concentration of salmeterol xinafoate is around 0.025% w/v and the propellant is 1,1,1,2-tetrafluoroethane, an amount of ethanol of 14-16% w/w, especially around 15% w/w is particularly suitable. When the concentration of salmeterol (present as xinafoate) is around 0.025% w/v (based on weight of salmeterol base) and the propellant is 1,1,1,2-tetrafluoroethane, the amount of ethanol is preferably 20-23% w/w eg 22% w/w. The above ethanol concentrations are appropriate for salmeterol xinafoate in the form of Form I polymorph. A somewhat lower concentration would be expected to be necessary for the Form II polymorph.

When the concentration of R-salmeterol (present as xinafoate) is 0.04 w/v (based on weight of R-salmeterol base) and the propellant is 1,1,1,2-tetrafluoroethane, an amount of ethanol of 12-14% w/w eg 13% w/w is suitable. When the concentration of R-salmeterol (present as xinafoate) is 0.025 w/v (based on weight of R-salmeterol base) and the propellant is 1,1,1,2-tetrafluoroethane, an amount of ethanol of 9-11 % w/w eg 10% w/w is suitable. When the concentration of R-salmeterol (present as xinafoate) is 0.025 w/v (based on weight of R-salmeterol base) and the propellant is 1,1,1,2,3,3,3-heptafluoro-n-propane, an amount of ethanol of 13-15% w/w eg 14% w/w is suitable.

When the concentration of salmeterol (present as free base) is 0.05 w/w and the propellant is 1,1,1,2-tetrafluoroethane, an amount of ethanol of 4-10% preferably 4-6% w/w eg 6% w/w is suitable. When the concentration of salmeterol (present as free base) is 0.04 w/w and the propellant is 1,1,1,2-tetrafluoroethane, an amount of ethanol of 4-10% preferably 4-6% w/w eg 5% w/w is suitable. When the concentration of salmeterol (present as free base) is 0.025 w/v and the propellant is 1,1,1,2-tetrafluoroethane, an amount of ethanol of 3-10% preferably 3-5% w/w eg 4 or 5% w/w is suitable. When the concentration of salmeterol (present as free base) is 0.05 w/v and the propellant is 1,1,1,2,3,3,3-heptafluoro-n-propane, an amount of ethanol of 4-10% preferably 4-6% w/w eg 6% w/w is suitable. When the concentration of salmeterol (present as free base) is 0.04 w/v and the propellant is 1,1,1,2,3,3,3-heptafluoro-n-propane, an amount of ethanol of 4-10% preferably 4-6% w/w eg 5% w/w is suitable. When the concentration of salmeterol (present as free base) is 0.025 w/v and the propellant is 1,1,1,2,3,3,3-heptafluoro-n-propane, an amount of ethanol of 3-10% preferably 3-5% w/w eg 4 or 5% w/w is suitable.

The preferred concentration of salmeterol (as free base) in the formulation is 0.025-0.05% w/w. When 1,1,1,2-tetrafluoroethane is the propellant the preferred concentration of ethanol as solubilising agent in the formulation is 3-12% eg 3-10% more preferably 3-6% especially 4-6% w/w. When 1,1,1,2,3,3,3-heptafluoro-n-propane is the propellant the preferred concentration of ethanol as solubilising agent in the formulation 3-12% eg 3-10% more preferably 3-6% especially 4-6% w/w. Higher concentrations of ethanol in HFA134a and HFA227 such as 8-10% eg 10% w/w will generally be employed when it desired to employ glycerol as the low volatility component at a level of 0.8% or above eg 1% w/w or so in order to assure the solubilisation of the glycerol in the formulation. Lower concentrations of ethanol in HFA134a and HFA227 such as 4-6% eg 5% w/w will generally be employed when it desired to employ glycerol as the low volatility component at a level of around 0.5% w/w or when the low volatility component is polyethylene glycol (eg PEG200 or PEG400).

Formulations according to the invention will preferably contain salmeterol or a salt thereof as the only medicament. However formulations which contain medicaments in addition to salmeterol or a salt such as corticosteroids or anti-cholinergic compounds may also be contemplated.

Formulations according to the invention which are free of surfactants are preferred. Formulations according to the invention which are free of all excipients besides the solubilisation agent (eg ethanol), low volatility component (such as glycerol or polyethylene glycol) and the propellant are particularly preferred. However we have observed that solution formulations of salmeterol show a tendency to exhibit chemical degradation on storage. Without being limited by theory we believe that this chemical degradation may be due to acid catalysed dimerisation of the salmeterol. Thus it may be preferred to incorporate an agent in an amount capable of preventing chemical degradation of salmeterol in the formulation. For examples agents capable of preventing acid catalysed dimerisation include bases such as sodium or potassium hydroxide or sodium carbonate or an organic amine. It may be necessary also to incorporate a small quantity of water into the formulation eg 0.05-2% w/w water or more preferably 0.1-1% w/w water. Chemical degradation may also be promoted by oxidation eg arising from trace amounts of peroxide present in valve components (such as peroxide cured rubbers) or excipients. Preferably peroxide contamination will be avoided eg by use of appropriately cleansed valve components and the like. Alternatively an anti-oxidant may be employed (preferably one which is not an acid). Formulations according to the invention which are free of all excipients besides the solubilisation agent (eg ethanol), low volatility component (such as glycerol or polyethylene glycol, the agent capable of preventing chemical degradation of salmeterol and any water in the formulation and the propellant are also preferred.

The pharmaceutical composition according to the present invention may be filled into canisters suitable for delivering pharmaceutical aerosol formulations. Canisters generally comprise a container capable of withstanding the vapour pressure of the HFA propellant, such as plastic or plastic-coated glass bottle or preferably a metal can, for example an aluminium can which may optionally be anodised, lacquer-coated and/or plastic-coated, which container is closed with a metering valve. Canisters may be coated with a polymer as described in WO 96/32151, for example, a blend of polyethersulphone (PES) and polytetrafluoroethylene (PTFE). Another polymer for coating that may be contemplated is FEP (fluorinated ethylene propylene). The metering valves are designed to deliver a metered amount of the formulation per actuation and incorporate a gasket to prevent leakage of propellant through the valve. The gasket may comprise any suitable elastomeric material such as for example low density polyethylene, chlorobutyl, black and white butadiene-acrylonitrile rubbers, butyl rubber, neoprene, EPDM (a polymer of ethylenepropylenediene monomer) (eg as described in WO95/02651) and TPE (thermoplastic elastomer; eg as described in W092/11190). EPDM and TPE rubbers are preferred. EPDM rubbers are particularly preferred. Suitable valves are commercially available from manufacturers well known in the aerosol industry, for example, from Valois, France (eg. DF10, DF30, DF60), Bespak plc, UK (eg. BK300, BK356, BK357) and 3M-Neotechnic Ltd, UK (eg. Spraymiser™). The DF31 valve of Valois, France is also suitable.

Valve seals, especially the gasket seal, and also the seals around the metering chamber, will preferably be manufactured of a material which is inert to and resists extraction into the contents of the formulation, especially when the contents include ethanol.

Valve materials, expecially the material of manufacture of the metering chamber, will preferably be manufactured of a material which is inert to and resists distortion by contents of the formulation, especially when the contents include ethanol. Particularly suitable materials for use in manufacture of the metering chamber include polyesters eg polybutyleneterephthalate (PBT) and acetals, especially PBT.

Materials of manufacture of the metering chamber and/or the valve stem may desirably be fluorinated, partially fluorinated or impregnated with fluorine containing substances in order to resist drug deposition.

Valves which are entirely or substantially composed of metal components (eg Spraymiser, 3M-Neotechnic) are especially suitable for use according to the invention.

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto an aluminium can to form an empty canister. The medicament is added to a charge vessel and a mixture of ethanol, low volatility component and liquefied propellant is pressure filled through the charge vessel into a manufacturing vessel. An aliquot of the formulation is then filled through the metering valve into the canister.

In an alternative process, an aliquot of the liquified formulation is added to an open canister under conditions which are sufficiently cold that the formulation does not vaporise, and then a metering valve crimped onto the canister.

In an alternative process, an aliquot of medicament dissolved in the solubilising agent and any low-volatility component is dispensed into an empty canister, a metering valve is crimped on, and then the propellant is filled into the canister through the valve.

Typically, in batches prepared for pharmaceutical use, each filled canister is check-weighed, coded with a batch number and packed into a tray for storage before release testing.

Each filled canister is conveniently fitted into a suitable channelling device prior to use to form a metered dose inhaler for administration of the medicament into the lungs or nasal cavity of a patient. Suitable channelling devices comprise, for example a valve actuator and a cylindrical or cone-like passage through which medicament may be delivered from the filled canister via the metering valve to the nose or mouth of a patient eg. a mouthpiece actuator.

In a typical arrangement the valve stem is seated in a nozzle block which has an orifice leading to an expansion chamber. The expansion chamber has an exit orifice which extends into the mouthpiece. Actuator (exit) orifice diameters in the range 0.15-0.45mm especially 0.2-0.45mm are generally suitable eg 0.25, 0.30, 0.33 or 0.42mm. 0.22mm is also suitable. We have found that it is advantageous to use a small diameter eg 0.25mm or less, particularly 0.22mm since this tends to result in a higher FPM and lower throat deposition. 0.15mm is also particularly suitable. The dimensions of the orifice should not be so small that blockage of the jet occurs.

Actuator jet lengths are typically in the range 0.30-1.7mm eg 0.30, 0.65 or 1.50mm. Smaller dimensions are preferred eg 0.65mm or 0.30mm.

For the avoidance of water ingress into the formulation it may be desired to overwrap the MDI product in a flexible package capable of resisting water ingress and capable of permitting absorption or release of any propellant which may leak from the canister. It may also be desired to incorporate a desiccant within the packaging. Example overwraps are described in US Patent 6119853.

Metered dose inhalers are designed to deliver a fixed unit dosage of medicament per actuation or 'puff', for example in the range of 10 to 5000 µg medicament per puff.

Administration of medicament may be indicated for the treatment of mild, moderate or severe acute or chronic symptoms or for prophylactic treatment. Treatment may be of asthma, chronic obstructive pulmonary disease (COPD) or other respiratory disorder. It will be appreciated that the precise dose administered will depend upon the age and condition of the patient, the quantity and frequency of administration will ultimately be at the discretion of the attendant physician. Typically, administration may be one or more times, for example from 1 to 8 times per day, giving for example 1,2,3 or 4 puffs each time. The preferred treatment regime is 2 puffs of 25µg/puff salmeterol, 2 times per day.

The filled canisters and metered dose inhalers described herein comprise further aspects of the present invention.

A still further aspect of the present invention comprises a method of treating respiratory disorders such as, for example, asthma or chronic obstructive pulmonary disease (COPD), which comprises administration by inhalation of an effective amount of a formulation herein before described.

A further aspect of the present invention comprises the use of a formulation herein before described in the manufacture of a medicament for the treatment of respiratory disorders, eg. asthma or chronic obstructive pulmonary disease (COPD).

As mentioned above the advantages of the invention in some or all of its embodiments include the fact that formulations according to the invention may be more environmentally friendly, more stable, less susceptible to Oswald ripening or drug deposition onto internal surfaces of a metered dose inhaler, have better dosing uniformity, deliver a higher FPM, give lower throat deposition, be more easily or economically manufactured, or may be otherwise beneficial relative to known formulations.

The invention is illustrated with reference to the following examples:

In the examples salmeterol xinafoate was used as the Form I polymorph (obtained by crystallisation from methanolic solution in isopropanol. R-salmeterol xinafoate was obtained by crystallisation from diethylether. Salmeterol base was obtained by crystallisation from ethyl acetate.

### Examples 1-3

| Formulations may be prepared with composition as follows: | |
|---|---|
| Salmeterol xinafoate: | 0.05% w/v |
| Ethanol: | 30% w/w |
| Glycerol: | 1.3% w/w |
| 1,1,1,2-tetrafluoroethane: | to 100% |

This solution formulation may be filled into an aluminium canister under pressure and fitted with a metering valve having a 50 µl metering chamber.

| | |
|---|---|
| Salmeterol xinafoate | 0.04% w/v |
| Ethanol | 24% w/w |
| Glycerol | 1.3% w/w |
| 1,1,1,2-tetrafluoroethane | to 100% |

This solution formulation may be filled into an aluminium canister under pressure and fitted with a metering valve having a 63 µl metering chamber.

| | |
|---|---|
| Salmeterol xinafoate | 0.025% w/v |
| Ethanol | 15% w/w |
| Glycerol | 1.3% w/w |
| 1,1,1,2-tetrafluoroethane | to 100% |

This solution formulation may be filled into an aluminium canister under pressure and fitted with a metering valve having a 100 µl metering chamber.

### Example 4

A formulation was prepared with compositions as follows:

| | |
|---|---|
| Salmeterol (as xinafoate) | 0.04% w/v (based on weight of salmeterol base) |
| Ethanol | 37% w/w |
| Glycerol | 1.0% w/w |
| 1,1,1,2-tetrafluoroethane | to 100% |

This solution formulation was filled into an aluminium canister (120 actuations/canister; overage of 40 actuations) under pressure and fitted with a metering valve (Valois DF60) having metering chamber of volume 63 µl. This formulation is suitable for delivering 25 µg salmeterol per actuation.

### Example 5

A formulation was prepared with compositions as follows:

| | |
|---|---|
| Salmeterol (as xinafoate) | 0.025% w/v (based on weight of salmeterol base) |
| Ethanol | 22% w/w |
| 1,1,1,2-tetrafluoroethane | to 100% |

This solution formulation was filled into an aluminium canister (120 actuations/canister; overage of 40 actuations) under pressure and fitted with a metering valve (Valois DF60) having metering chamber of volume 100 µl. This formulation is suitable for delivering 25 µg salmeterol per actuation.

### Example 6

A formulation was prepared with compositions as follows:

| | |
|---|---|
| Salmeterol (as xinafoate) | 0.04% w/v (based on weight of salmeterol base) |
| Ethanol | 37% w/w |
| 1,1,1,2-tetrafluoroethane | to 100% |

This solution formulation was filled into an aluminium canister (120 actuations/canister; overage of 40 actuations) under pressure and fitted with a metering valve (Valois DF60) having metering chamber of volume 63 µl. This formulation is suitable for delivering 25 µg salmeterol per actuation.

### Example 7

A formulation was prepared with compositions as follows:

| | |
|---|---|
| Salmeterol (as xinafoate) | 0.025% w/v (based on weight of salmeterol base) |
| Ethanol | 22% w/w |
| Glycerol | 1.0% w/w |
| 1,1,1,2-tetrafluoroethane | to 100% |

This'solution formulation was filled into an aluminium canister (120 actuations/canister; overage of 40 actuations) under pressure and fitted with a metering valve (Valois DF60) having metering chamber of volume 100 µl. This formulation is suitable for delivering 25 µg salmeterol per actuation.

### Examples 8 and 9

Formulations were prepared with compositions as follows:

| | | |
|---|---|---|
| R-Salmeterol (as xinafoate) | 0.025% w/v* | 0.025% w/v* |
| Ethanol | 10% w/w | 10% w/w |
| Glycerol | 0% | 0.5% w/w |
| 1,1,1,2-tetrafluoroethane | to 100% | to 100% |

| | | |
|---|---|---|
| (*based on weight of R-salmeterol base) | | |

### Examples 10, 11 and 12

Formulations were prepared with compositions as follows:

| | | | |
|---|---|---|---|
| R-Salmeterol (as xinafoate) | 0.025% w/v* | 0.025% w/v* | 0.025% w/v* |
| Ethanol | 14% w/w | 14% w/w | 14% w/w |
| Glycerol | 0% | 0.5% w/w | 1% w/w |
| 1,1,1,2,3,3,3-heptafluoro-n-propane | to 100% | to 100% | to 100% |

| | | | |
|---|---|---|---|
| (*based on weight of R-salmeterol base) | | | |

The solution formulations of Examples 8 to 12 were filled into an aluminium canister (120 actuations/canister; overage of 40 actuations) under pressure and fitted with a metering valve (Valois DF60) having metering chamber of volume 100 µl. These formulation are suitable for delivering 25 µg R-salmeterol per actuation.

### Examples 13, 14 and 15

Formulations were prepared with compositions as follows:

| | | | |
|---|---|---|---|
| R-Salmeterol (as xinafoate) | 0.04% w/v* | 0.04% w/v* | 0.04% w/v* |
| Ethanol | 13% w/w | 13% w/w | 13% w/w |
| Glycerol | 0% | 0.5% w/w | 1% w/w |
| 1,1,1,2-tetrafluoroethane | to 100% | to 100% | to 100% |

| | | | |
|---|---|---|---|
| (*based on weight of R-salmeterol base) | | | |

The solution formulations of Examples 13, 14 and 15 were filled into aluminium canisters (120 actuations/canister; overage of 40 actuations) under pressure and fitted with a metering valve (Valois DF60) having metering chamber of volume 63 µl. These formulations are suitable for delivering 25 µg R-salmeterol per actuation.

### Examples 16 , 17, 18 and 19

Formulations were prepared with compositions as follows:

| | | | | |
|---|---|---|---|---|
| Salmeterol base | 0.025% w/v | 0.025% w/v | 0.04% w/v | 0.04% w/v |
| Ethanol | 5% w/w | 5% w/w | 10% w/w | 10% w/w |
| PEG200 | 0% | 0.5% w/w | 0% w/w | 0.5% |
| 1,1,1,2-tetrafluoroethane | to 100% | to 100% | to 100% | to 100% |

### Examples 20 , 21, 22 and 23

Formulations were prepared with compositions as follows:

| | | | | |
|---|---|---|---|---|
| Salmeterol base | 0.025% w/v | 0.025% w/v | 0.04% w/v | 0.04% w/v |
| Ethanol | 5% w/w | 5% w/w | 10% w/w | 10% w/w |
| PEG200 | 0% | 0.5% w/w | 0% w/w | 0.5% |
| 1,1,1,2,3,3,3-heptafluoro-n-propane | to 100% | to 100% | to 100% | to 100% |

### Example 24

A formulation was prepared with composition as follows:

| | |
|---|---|
| Salmeterol base | 0.04% w/v |
| Ethanol | 10% w/w |
| Glycerol | 0.5% |
| 1,1,1,2,3,3,3-heptafluoro-n-propane | to 100% |

### Examples 25, 26 and 27

Formulations were prepared with compositions as follows:

| | | | |
|---|---|---|---|
| Salmeterol base | 0.04% w/v | 0.04% w/v | 0.04% w/v |
| Ethanol | 10% w/w | 10% w/w | 10% w/w |
| Glycerol | 0.5% w/w | 0% | 0% |
| PEG400 | 0% | 0.5% w/w | 0% |
| Propylene glycol | 0% | 0% | 0.5% w/w |
| 1,1,1,2-tetrafluoroethane | to 100% | to 100% | to 100% |

The solution formulations of Examples 16, 17, 20 and 21 were filled into aluminium canisters (120 actuations/canister; overage of 40 actuations) under pressure and fitted with a metering valve (Valois DF60) having metering chamber of volume 100 µl. These formulations are suitable for delivering 25 µg salmeterol per actuation.

The solution formulations of Examples 18, 19 and 22-27 were filled into aluminium canisters (120 actuations/canister; overage of 40 actuations) under pressure and fitted with a metering valve (Valois DF60) having metering chamber of volume 63 µl. These formulations are suitable for delivering 25 µg salmeterol per actuation.

### Andersen Cascade Impaction Data

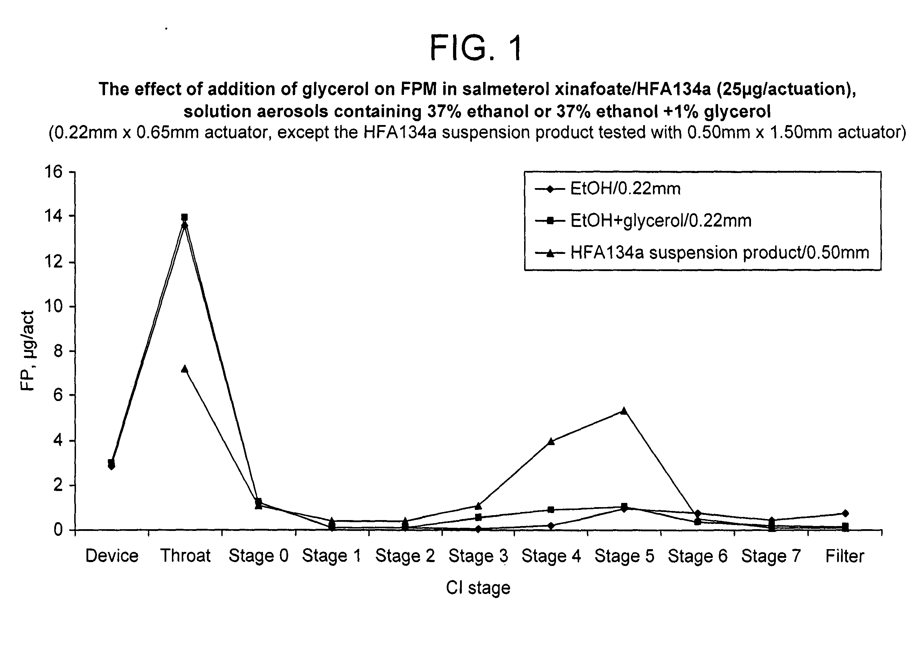

Formulations as described in Examples 4 and 6 were profiled using an Andersen Cascade Impactor, using a 0.22mm (orifice) x 0.65mm (jet length) actuator from Bespak (BK621 variant). Testing was performed on canisters at "beginning of use" (BoU) and delivered drug from 10 actuations was collected in the instrument after 4 priming actuations were fired to waste. Results are shown in Table 1 and Figure 1. For comparison data from a product consisting of particulate salmeterol xinafoate suspensed in HFA134a (excipient free) (25 µg per actuation) is also shown.

Further similar tests were performed as follows:

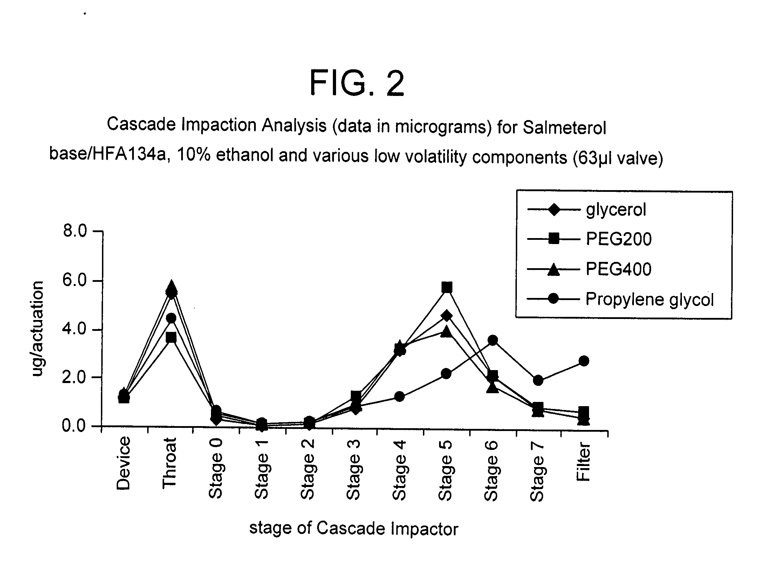

On Examples 19, 25, 26 and 27 results of which are shown in Table 2 and Figure 2;

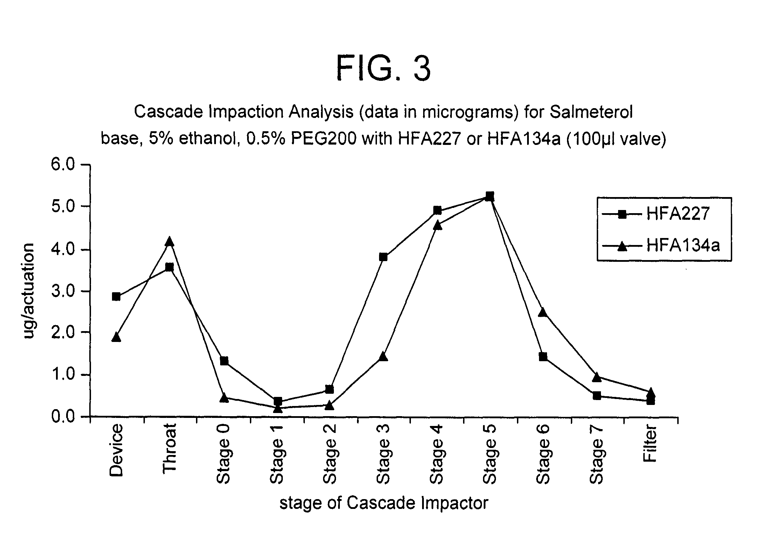

On Examples 17 and 21 results of which are shown in Table 3 and Figure 3;

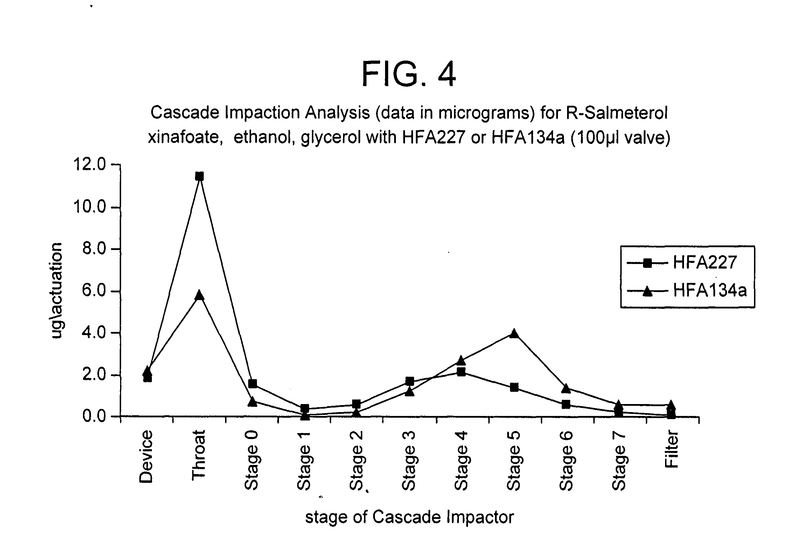

On Examples 9 and 12 results of which are shown in Table 4 and Figure 4; and

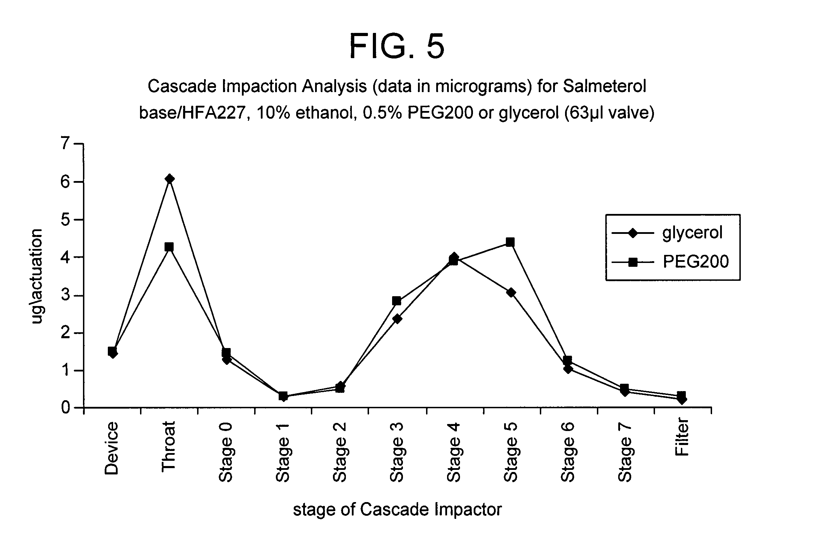

On Examples 23 and 24 results of which are shown in Table 5 and Figure 5;

### Brief Description of the Tables:

Table 1: Cascade Impaction analysis of salmeterol xinafoate/HFA134a solution aerosols containing 37% ethanol with and without 1% glycerol
Table 2: Cascade Impaction analysis of salmeterol base/HFA134a solution aerosols containing 10% ethanol with 0.5% of various low volatility components
Table 3: Cascade Impaction analysis of salmeterol base solution aerosols containing 5% ethanol and 0.5% PEG200 in HFA134a or HFA227
Table 4: Cascade Impaction analysis of R-salmeterol xinafoate solution aerosols containing 10% ethanol and 0.5% glycerol in HFA134a or 14% ethanol and 1% glycerol in HFA227
Table 5: Cascade Impaction analysis of salmeterol base/HFA227 solution aerosols containing 10% ethanol with 0.5% of various low volatility components
The above Tables show the Cascade Impaction analysis data in terms of absolute microgram quantities and percentages.

### Brief Description of the Figures:

Figure 1: Cascade Impaction analysis of salmeterol xinafoate/HFA134a solution aerosols containing 37% ethanol with and without 1% glycerol
Figure 2: Cascade Impaction analysis of salmeterol base/HFA134a solution aerosols containing 10% ethanol with 0.5% of various low volatility components
Figure 3: Cascade Impaction analysis of salmeterol base solution aerosols containing 5% ethanol and 0.5% PEG200 in HFA134a or HFA227
Figure 4: Cascade Impaction analysis of R-salmeterol xinafoate solution aerosols containing 10% ethanol and 0.5% glycerol in HFA134a or 14% ethanol and 1% glycerol in HFA227
Figure 5: Cascade Impaction analysis of salmeterol base/HFA227 solution aerosols containing 10% ethanol with 0.5% of various low volatility components
   The above Figures show the Cascade Impaction analysis data in terms of absolute microgram quantities.

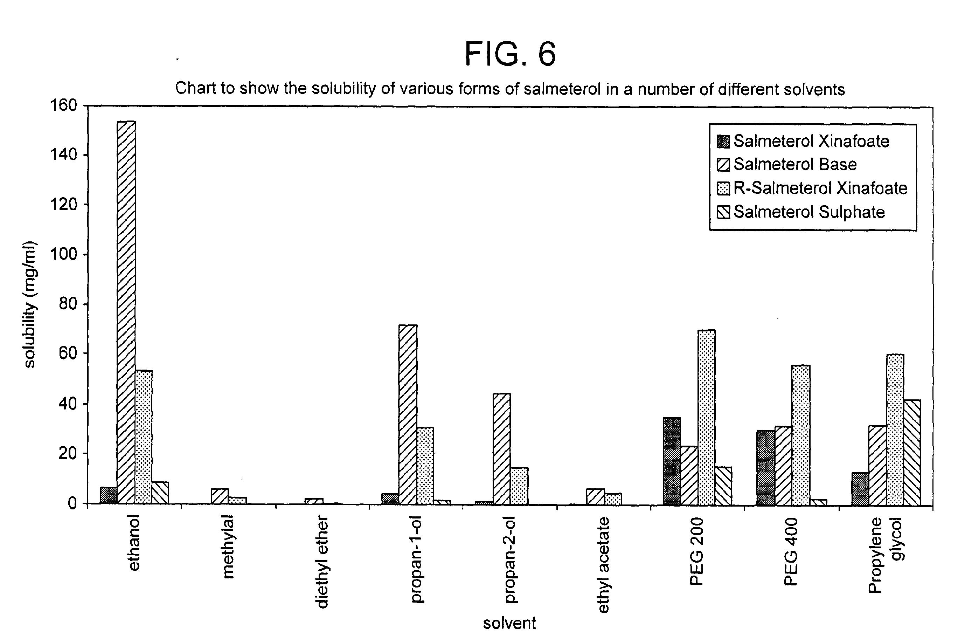

Figure 6: Chart to show the solubility of various forms of salmeterol in a number of different solvents.

From the Tables and Figures it may be deduced that exceptionally good data in terms of fine particle mass is obtained from the use of salmeterol base using ethanol as solubilising agent and HFA134a or HFA227 as propellant with glycerol or polyethylene glycol (PEG200, PEG400) as low volatility component. Use of PEG200 tended to result in particularly low throat deposition.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

Above mentioned patents and patent applications are hereinbefore incorporated by reference.

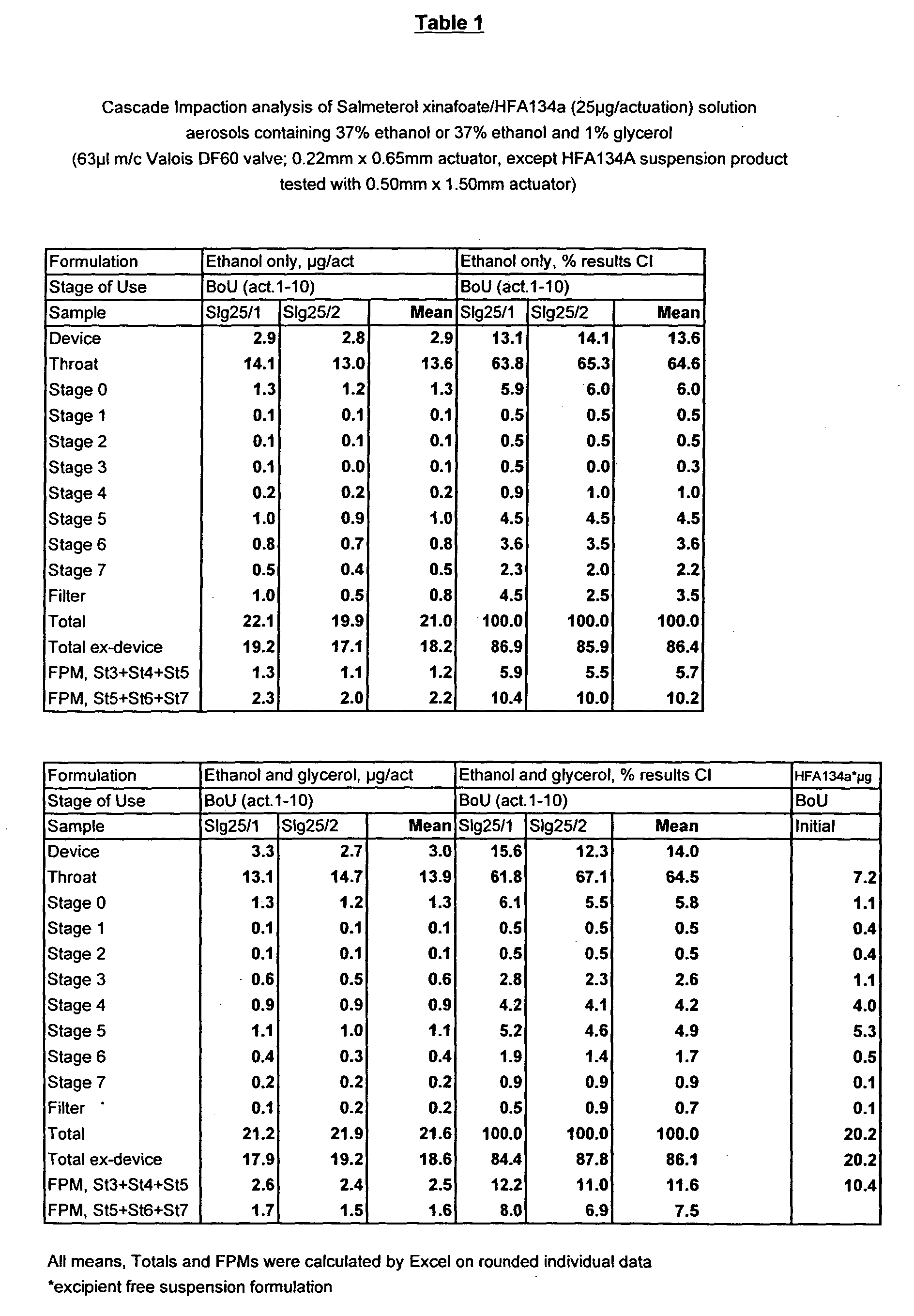

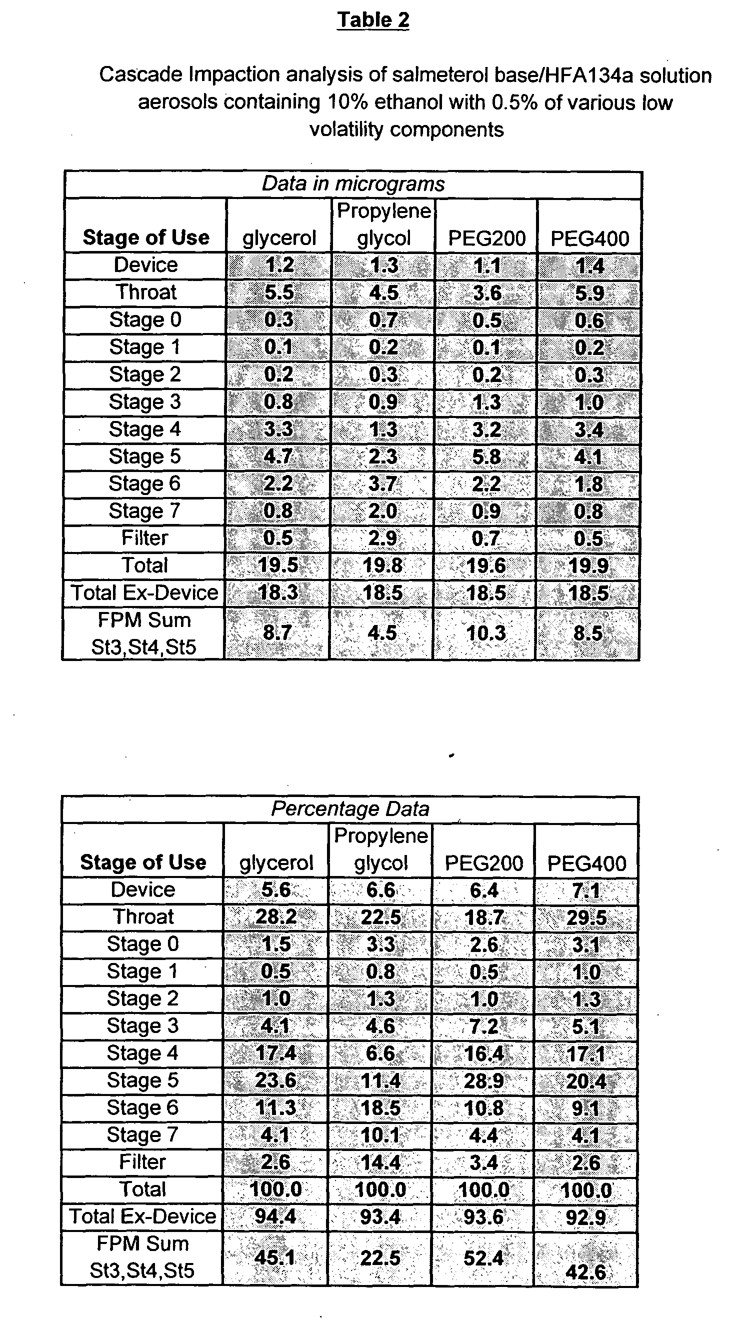

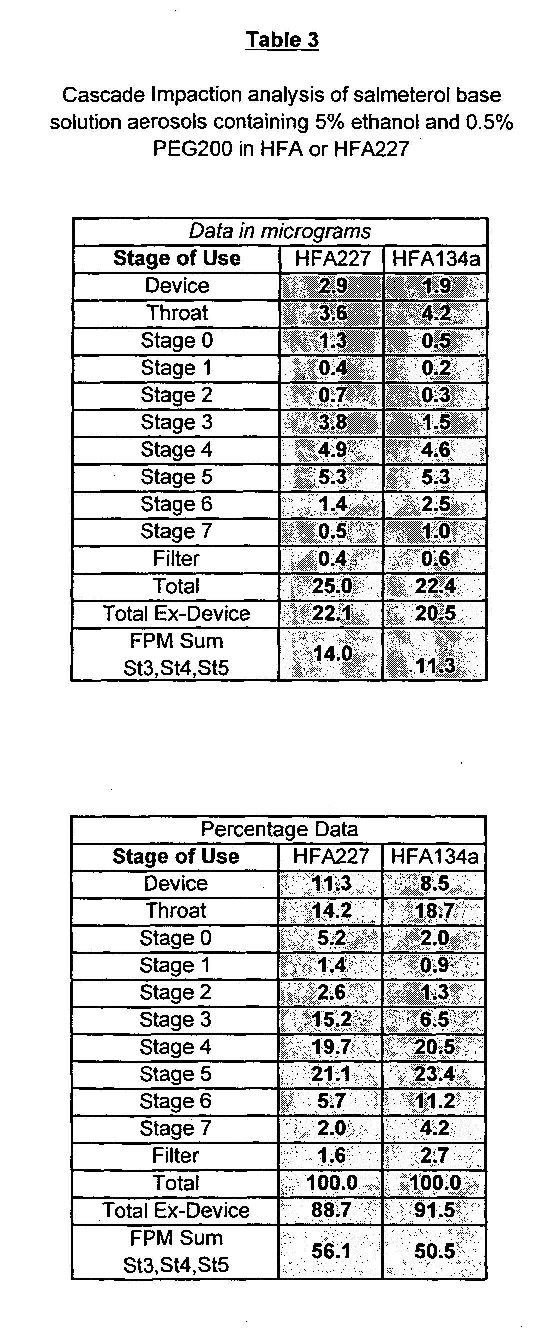

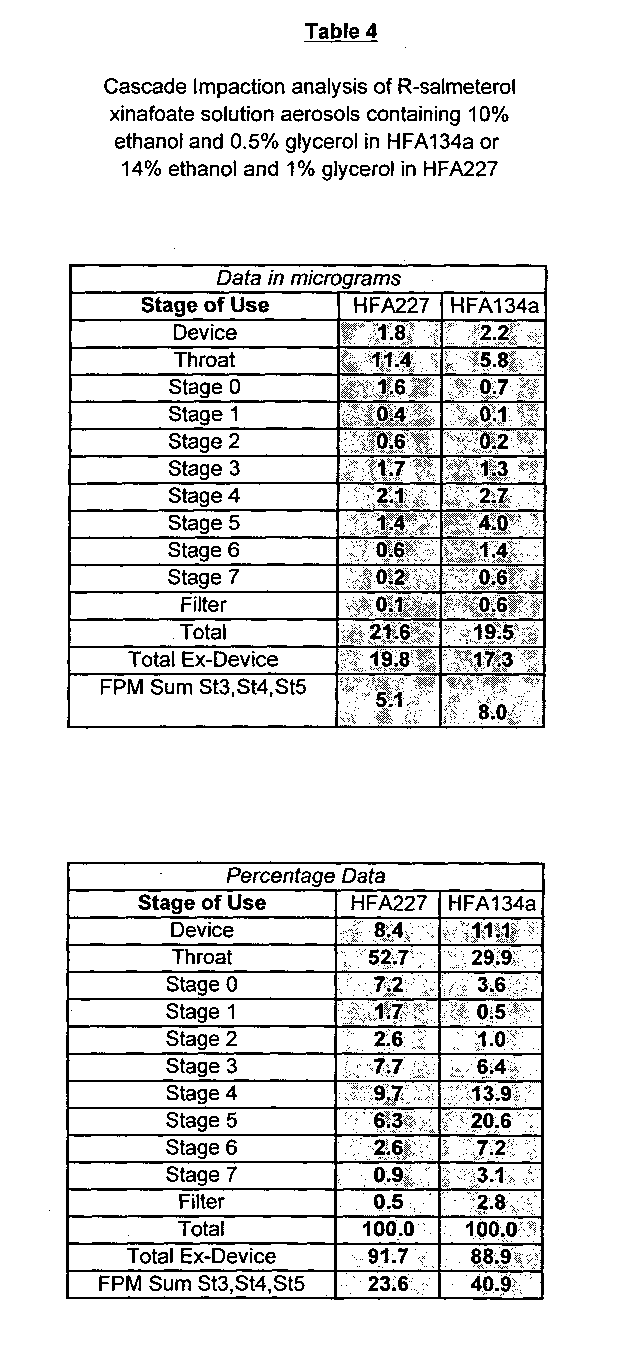

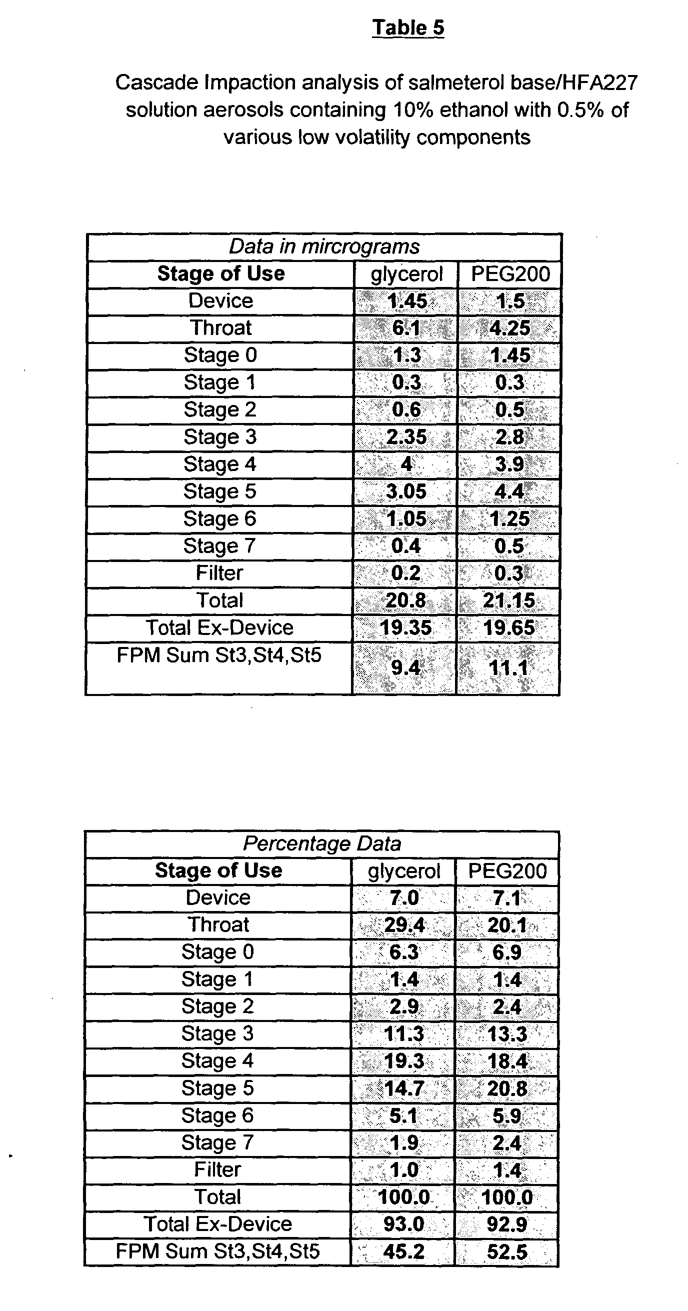

## Claims

1. A pharmaceutical aerosol formulation which comprises:
(i) salmeterol or a pharmaceutically acceptable salt thereof and
(ii) a hydrofluoroalkane (HFA) propellant,
**characterised in that** the salmeterol or pharmaceutically acceptable salt thereof is completely dissolved in the formulation.

2. A formulation according to claim 1 which comprises:
(i) salmeterol or a pharmaceutically acceptable salt thereof;
(ii) a hydrofluoroalkane (HFA) propellant;
(iii) a low volatility component to increase the mass median aerodynamic diameter (MMAD) of the aerosol particles on actuation of the inhaler; and
(iv) a solubilisation agent in sufficient quantity to solubilise the salmeterol or pharmaceutically acceptable salt thereof in the formulation.

3. A formulation according to claim 1 or claim 2 wherein the hydrofluoroalkane (HFA) propellant is 1,1,1,2-tetrafluoroethane (HFA134a).

4. A formulation according to claim 1 or claim 2 wherein the hydrofluoroalkane (HFA) propellant is 1,1,1,2,3,3,3-heptafluoro-n-propane (HFA227).

5. A formulation according to any one of claims 1 to 4 containing a low volatility component which is glycerol.

6. A formulation according to any one of claims 1 to 4 containing a low volatility component which is polyethylene glycol.

7. A formulation according to claim 6 wherein the low volatility component is PEG200.

8. A formulation according to any one of claims 1 to 7 containing ethanol as solubilising agent in sufficient quantity to solubilise the salmeterol or pharmaceutically acceptable salt thereof in the formulation.

9. A formulation according to claim 8 wherein the concentration of ethanol is 5 to 30% w/v.

10. A formulation according to claim 8 wherein the concentration of ethanol is 5 to 12% w/v.

11. A formulation according to any one of claims 1 to 10 wherein salmeterol is present as salmeterol base.

12. A formulation according to any one of claims 1 to 9 wherein salmeterol is present as the xinafoate salt.

13. A formulation according to claim 12 wherein salmeterol xinafoate is present in the form of its Form II polymorph.

14. A formulation according to claims 1 to 13 wherein the concentration of salmeterol expressed as weight of xinafoate is 0.02-0.05% w/v.

15. A formulation according to claim 11 wherein the concentration of salmeterol base is 0.025-0.05% w/v.

16. A formulation according to any one of claims 1 to 15 wherein salmeterol is present as R-salmeterol.

17. A formulation according to any one of claims 1 to 16 which contains a low volatility component at between 0.5 and 3% (w/w).

18. A formulation according to claim 17 which contains between 1.0 and 1.6% (w/w) of the low volatility component.

19. A formulation according to claim 17 which contains 1.0% (w/w) of the low volatility component.

20. A formulation according to claim 17 which contains between 0.5 and 1.0% (w/w) of the volatility component.

21. A formulation according to claims 1 which comprises:
(i) 0.025-0.05% w/w salmeterol base;
(ii) 1,1,1,2-tetrafluoroethane, 1,1,1,2,3,3,3-heptaftuoro-n-propane or a mixture thereof as propellant;
(iii) 0.5-1% of a low volatility propellant selected from glycerol and polyethylene glycol; and
(iv) 3-12% ethanol as solubilising agent.

22. A formulation according to claim 21 wherein the low volatility component is PEG200.

23. A formulation according to any one of claims 1 to 22 further comprising a compound capable of preventing chemical degradation of salmeterol in the formulation.

24. A formulation according to any one of claims 1 to 23 which further comprises water.

25. A formulation according to claim 24 which comprises from about 0.05 to about 2% (w/w) water.

26. A canister comprising a metering valve and containing a pharmaceutical aerosol formulation according to any one of claims 1 to 25.

27. A metered dose inhaler which comprises a canister as claimed in claim 26 fitted into a suitable channelling device.

28. Use of a pharmaceutical aerosol formulation according to any one of claims 1 to 25 in the manufacture of a medicament for the treatment of respiratory disorders, eg. asthma or chronic obstructive pulmonary disease (COPD).
